# EUROPEAN PATENT APPLICATION

(11) **EP 1 975 277 A1**
(43) Date of publication of application: **01.10.2008**
(21) Application number: 08005180.8
(22) Date of filing: 19.03.2008
(51) Int. Cl.: C23C 22/64, C23C 22/68, A61L 27/06

(54) **Surface treatment method of titanium or titanium alloy**

(30) Priority: 27.03.2007 JP 2007080432
(71) Applicant: GC Corporation, Itabashi-ku Tokyo 174-8585 (JP)
(72) Inventor: Ishikawa Kunio, Kasuya-gun Fukuoka (JP); Yamamoto Katsushi, Tokyo (JP)
(74) Representative: Müller-Boré & Partner Patentanwälte

(57) **Abstract**

An objective of the present invention is to provide a surface treatment method of titanium or a titanium alloy with a shortened time for depositon of apatite on a material surface after the treatment so that early-stage osseointegration can be expected. This surface treatment method of titanium or a titanium alloy has a higher effect to form a calcium layer than a conventional method using calcium chloride. In the surface treatment method, titanium or a titanium alloy is contacted with a solution of calcium salt except calcium halide being dissolved or suspended.

## Description

The present invention relates to a surface treatment method which is proper for an implant made of titanium or a titanium alloy implanted in a living body.

In the field of an implant, titanium or a titanium alloy has beenmainlyused as a raw material. The reason of this is as follows. Titanium or a titanium alloy is covered with an oxide film mainly including titanium oxide, and this film becomes a passivating film against corrosion in body fluid so as to suppress corrosion of titanium or a titanium alloy. Further, since this film has high affinity with respect to a bone, the osseointegration period can be shortened.

As a method for shortening the osseointegration period, for example, Unexamined Japanese Patent Publication No. 8-182755 discloses a method of thermal spraying or sputtering in which the surface of titanium or a titanium alloy is covered with a hydroxyl apatite (it will be called just "apatite" below) which is a main inorganic component of a bone. However, since the surface of a metal, that is titanium or titanium alloy, is covered with a ceramic, that is an apatite layer, the bond force between both materials decreases with the elapse of time. Particularly, when five or more years have elapsed, there is a problem in poor prognosis.

As other methods, Unexamined Japanese Patent Publication No. 8-299429 discloses a method in which titanium or a titanium alloy is dipped in an alkaline or hydrogen peroxide solution, and Unexamined Japanese Patent Publication No. 9-94260 discloses a method in which titanium or a titanium alloy is dipped in a solution including calcium ions at a temperature from a room temperature to a boiling point. When a titanium material treated with these methods is stored in a simulated body fluid including calcium or phosphorus ions, a deposited apatite on a surface of the titanium material canbe confirmed, and thus there is an advantage that a large-scale treatment apparatus is not necessary. However, these treatments have a problem that an efficiency to deposit apatite on the implant surface is low.

In order to solve the above-described problems, present inventors developed a surface treatment method as disclosed in Unexamined Japanese Patent Publication No. 2006-102212 in which a surface of an implant made of titanium or a titanium alloy is dipped in a solution including calcium chloride. In this method, early-stage osseointegration in a living body was expected by forming of a calcium layer which becomes core for forming of apatite so as to shorten a time for deposition of apatite. However, this method has a fault that calcium chloride corrodes a titanium oxide layer on an implant surface, and thus there is a problem that calcium is not formed so efficiently as expected.

An objective of the present invention is to provide a surface treatment method of titanium or a titanium alloy without having the conventional problem that the bond force between a titanium material and apatite decreases with the elapse of time in a case of thermal spray coating. The present invention also provides the surface treatment method capable of shortening a time for depositing apatite on the titanium material surface after the treatment so that early-stage osseointegration can be expected. This surface treatment method of titanium or a titanium alloy has a higher effect to form a calcium layer than a conventional method using calcium chloride.

The present inventors carried out earnest works to solve the above-described problems and, as a result of this, they found out the followings to complete the present invention. When titanium or a titanium alloy is treated with calcium chloride, a titanium oxide layer is corroded by an operation of chlorine (halogen) contained in calcium chloride. However, when titanium or a titanium alloy is treated with calcium salt except calcium halide, corrosion of a titanium oxide layer is suppressed. Further, a calcium layer can be formed efficiently on a so treated titanium material. Therefore, osseointegration in a shorter period can be expected.

That is, an aspect of the present invention is a surface treatment method of titanium or a titanium alloy that titanium or a titanium alloy is contacted with a solution in which calcium salt except calcium halide is dissolved or suspended. When the condition of contacting of titanium or a titanium alloy with the solution in which calcium salt except calcium halide is dissolved or suspended is a hydrothermal condition, a higher treatment effect can be obtained. Further, calcium salt except calcium halide is preferably one or more kinds selected from calcium oxide, calcium hydroxide, calcium carbonate, calcium borate, calcium sulfate, calcium hydride, calcium silicate, calcium carbide, and calcium silicide, and/or calcium phosphate, and/or organic acid calcium, and/or metal calcium.

The surface treatment method of titanium or a titanium alloy according to the present invention has no conventional problem in case of thermal spraying that the bond force between a titanium material and apatite decreases with the passage of time and can shorten a time for deposition of apatite on the material surface after the treatment. Thus, early-stage osseointegration can be expected. Further, this surface treatment method is little affected by the problem that a calcium layer is influenced by corrosion of a titanium oxide layer in a conventional method using a calcium chloride, and thus is excellent.

In a surface treatment method of titanium or a titanium alloy according to the present invention, titanium or a titanium alloy is contacted with the solution in which calcium salt except calcium halide is dissolved or suspended. At this time, it is preferable that titanium or a titanium alloy is treated under a hydrothermal condition in view of efficiency. More particularly, it is preferable that an implant made of titanium or a titanium alloy is dipped in the solution at 120°C to 250°C for 1 to 48 hours. In the present invention, treatment temperature is more important than pressure, and the pressure becomes equilibrium pressure at the desired temperature. When titanium or a titanium alloy is treated under a hydrothermal condition, a treatment time is shorter than that of a surface treatment which is done at temperature from a room temperature to a boiling point at atmospheric pressure. Further, a surface treatment can be carried out more accurately and efficiently if the treatment time is the same. Furthermore, as a method of contacting, a steam contact or dipping is preferable since those are easy and efficient.

When the treatment temperature is less than 120°C under a hydrothermal condition, the treatment time is long or an oxide film to be obtained is thinner than that obtained with a treatment at 120°C or more. Thus, the effect of the surface treatment may decrease. On the other hand, the treatment temperature of more than 250°C is effective but the treatment itself may be dangerous. When the contact time is less than 1 hour, a significant effect for apatite deposition at the time of dipping in a simulated body fluid cannot be obtained in comparison with the case of an untreated material. On the other hand, when the contact time is more than 48 hours, an apatite layer becomes too thick and thus the layer may be peeled after embedding.

In the surface treatment method of titanium or a titanium alloy according to the present invention, concentration of a solution of calcium salt except calcium halide is preferably 5 to 100m mol/L. When the concentration is less than 5m mol/L, an efficiency of the treatment may decrease. When the concentration is more than 100m mol/L, the efficiency does not remarkably increase.

Examples of the calcium salt except calcium halide to be used in the present invention are one or more kinds selected from calcium oxide, calcium hydroxide, calcium carbonate, calcium borate, calcium sulfate, calcium hydride, calcium silicate, calcium carbide and calcium silicide, and/or calcium phosphate (e.g., hydroxyapatite, carbonate group-containing apatite, tricalcium phosphate, tetracalcium phosphate, calcium hydrogen phosphate, calcium octaphosphate), and/or organic acid calcium (e.g., calcium lactate, calcium citrate, calcium tartrate, calcium benzoate, calcium formate, calcium gluconate), and/or metal calcium.

The surface treatment method of titanium or a titanium alloy according to the present invention will be described below with reference to Examples and Comparative examples. However, the present invention is not limited to these Examples.

### <Example 1>

A test sample was produced by washing a surface polished pure titanium plate (JIS2 class) having a diameter of 6mm and a thickness of 2mm, dipping it in a calcium oxide aqueous solution of 10m mol/L at 200°C for 24 hours, and thereafter washing and drying it.

### <Example 2>

A test sample was produced by washing a surface polished pure titanium plate (JIS2 class) having a diameter of 6mm and a thickness of 2mm, dipping it in a calcium lactate aqueous solution of 10m mol/L at 200°C for 24 hours, and thereafter washing and drying it.

### <Comparative example 1>

A test sample was produced by washing a surface polished pure titanium plate (JIS2 class) having a diameter of 6mm and a thickness of 2mm, dipping it in a calcium chloride aqueous solution of 10m mol/L at 200°C for 24 hours, and thereafter washing and drying it.

### <Comparative example 2>

A test sample was produced by washing a surface polished pure titanium plate (JIS2 class) having a diameter of 6mm and the thickness of 2mm, dipping it in distilled water at 200°C for 24 hours, and thereafter washing and drying it.

### <Comparative example 3>

A test sample was produced by washing a surface polished pure titanium plate (JIS2 class) having a diameter of 6 mm and the thickness of 2mm, and drying it.

These samples were dipped in a simulatedbody fluid at 37°C for 7 days, and the calcium concentration and phosphorous concentration in the simulated body fluid after 7 days were measured by absorbance measurement with a coloring reagent. These results were shown in Table 1.

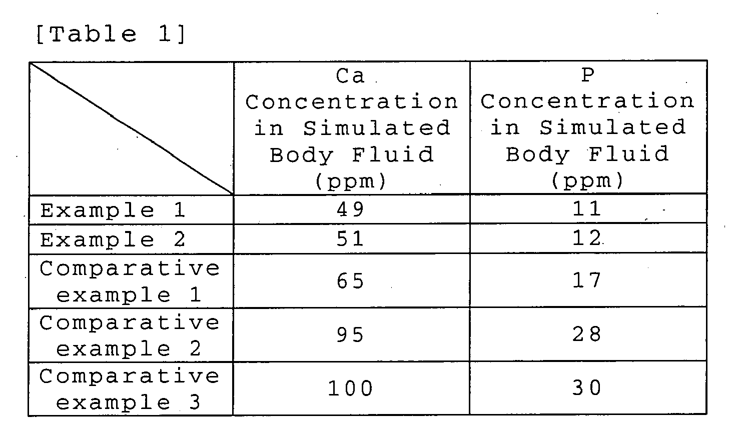

As for the samples of Examples 1 and 2 subjected to a surface treatment by the surface treatment method of titanium or a titanium alloy according to the present invention, the concentrations of Ca and P in a simulated body fluid decreases in comparison with those of the samples of Comparative examples 1 to 3. Thus, more amounts of Ca and P are taken into the sample surfaces.

## Claims

1. A surface treatment method of titanium and a titanium alloy, the method comprising:
contacting of titanium or a titanium alloy to a solution in which a calcium salt except calcium halide is dissolved or suspended.

2. The surface treatment method of titanium and a titanium alloy as claimed in claim 1,
wherein the condition of contacting of titanium or a titanium alloy with the a solution in which the calcium salt except calcium halide is dissolved or suspended is a hydrothermal condition.

3. The surface treatment method of titanium and a titanium alloy as claimed in claim 1 or 2,
wherein the calcium salt except calcium halide is one or more kinds selected from calcium oxide, calcium hydroxide, calcium carbonate, calcium borate, calcium sulfate, calcium hydride, calcium silicate, calcium carbide, and calcium silicide, and/or calcium phosphate, and/or organic acid calcium, and/or metal calcium.
